# EUROPÄISCHE PATENTSCHRIFT

(11) **EP 1 232 732 B1**
(45) Veröffentlichungstag und Bekanntmachung des Hinweises auf die Patenterteilung: **19.09.2007**
(21) Anmeldenummer: 01124050.4
(22) Anmeldetag: 09.10.2001
(51) Int. Cl.: A61F 2/52

(54) **Symmetrische Brustprothese**
Symmetric breast prosthesis
Prothèse mammaire symétrique

(30) Priorität: 28.11.2000 DE 10059013
(43) Veröffentlichungstag der Anmeldung: 21.08.2002
(73) Patentinhaber: F + E Gesellschaft für Bekleidungsinnovation mbH & Co. KG, 83098 Brannenburg (DE)
(72) Erfinder: Weber-Unger, Georg, 6330 Kufstein (AT); Volk, Stephan, 83714 Miesbach (DE)
(74) Vertreter: Haug, Dietmar

(56) Entgegenhaltungen:
- EP-A- 0 384 951
- DE-A- 19 754 144
- DE-A- 19 838 428

## Beschreibung

Die Erfindung betrifft eine symmetrische Brustprothese zum Tragen in der linken oder rechten Büstenschale eines Büstenhalters oder Korseletts nach einer Brustamputation bei Frauen, mit einem Prothesenkörper, der eine die Form einer natürlichen Brust aufweisende Vorderseite, eine mit wenigstens einer länglichen Kerbe versehene konkave Rückseite und einen eine geschlossene Linie bildenden Prothesenrand hat, an dem die Vorderseite und die Rückseite jeweils enden, wobei der Prothesenkörper eine weich-elastische Silikonkautschukmasse aufweist, die in einen Beutel aus längs des Prothesenrandes dicht miteinander verbundenen, elastisch dehnbaren Kunststoffolien hohlraumfrei eingeschlossen ist.

Eine solche Brustprothese ist aus der DE 198 38 428 A1 bekannt. Sie hat eine symmetrische Dreiecksform und kann infolgedessen wahlweise als Ersatz für die rechte oder linke amputierte Brust getragen werden, wobei die Kerbe oder Kerben auf der Rückseite des Prothesenkörpers jeweils quer zur Körperlängsachse der Trägerin verläuft bzw. verlaufen. Obwohl diese Brustprothese einen fast vollkommenen Ausgleich für das entfernte Brustgewebe bis in den Achselbereich bietet, wird sie von Prothesenträgerinnen nicht immer als optimal empfunden, weil der seitliche Ausläufer zum Brustbein zu lang sein kann und dann stört, besonders wenn die Prothese in einem Bügel-Büstenhalter getragen wird.

Eine andere symmetrische Brustprothese, die wahlweise auf der linken oder rechten Brustseite getragen werden kann, hat einen tropfenförmigen Grundriß. Eine solche Brustprothese ist zum Beispiel dem DE-GM 69 26 549 entnehmbar. Sie eignet sich besonders für diejenigen Frauen, bei denen der obere Brustansatz erhalten werden konnte und ein Ausgleich für im Achselbereich wegoperiertes Gewebe notwendig ist. Sie wird auch manchmal so getragen, daß ihr Ausläufer zur Schulter hin weist, wenn beim oberen Brustansatz Gewebe entfernt worden ist. Aufgrund ihrer Tropfenform kann sie jedoch nicht gleichzeitig wegoperiertes Gewebe im Achselbereich und am oberen Brustansatz ausgleichen.

Es sind aber auch symmetrische Brustprothesen bekannt, die sowohl einen seitlichen Ausläufer als auch einen oberen Ausläufer haben, wobei die beiden Ausläufer gleich lang sind. Diese Brustprothesen haben annähernd eine Herzform und werden so getragen, daß ihre zwischen den beiden Ausläufern verlaufende Symmetrieebene einen Winkel von etwa 45° mit der Körperlängsachse der Trägerin einschließt, wobei beim linksseitigen wie auch beim rechtsseitigen Tragen der Brustprothese immer einer der Ausläufer zur Schulter und der jeweils andere Ausläufer zur Achselhöhle weist. Die bisher bekannten herzförmigen Brustprothesen entsprechen im Unterbrustbereich aber auch zum Brustbein hin nicht immer den natürlichen Gegebenheiten. Außerdem sind oft die Ausläufer zur Achsel hin zu kurz oder zur Schulter hin zu lang.

Die ideale Form der Brustprothese hatte bislang nur die asymmetrische Brustprothese die zwei Ausläufer aufweist, von denen der obere Ausläufer kleiner als der seitliche Ausläufer ist. Zwangsläufig sind asymmetrische Brustprothesen so ausgebildet, daß sie entweder nur auf der linken Körperseite oder nur auf der rechten Körperseite getragen werden können. Da die Formen für "linke" asymmetrische Brustprothesen verschieden von den Formen für "rechte" asymmetrische Brustprothesen sind, sind asymmetrische Brustprothesen in der Herstellung grundsätzlich teurer als symmetrische Brustprothesen, bei denen die Seite, auf der sie getragen werden, ja unabhängig von den bei der Herstellung verwendeten Formen ist. Da asymmetrische Brustprothesen entweder nur links oder nur rechts getragen werden können, ist auch ihre Lagerhaltung im Handel aufwendiger als die von symmetrischen Brustprothesen. Im Vergleich zu symmetrischen Brustprothesen müßten nämlich bei asymmetrischen Brustprothesen die doppelte Stückzahl auf Lager genommen werden, wenn die Versorgung von betroffenen Frauen mit symmetrischen oder asymmetrischen Brustprothesen gleichermaßen gewährleistet sein soll. Wenn Brustprothesen, wie üblich, in 10 bis 15 verschiedenen Größen angeboten werden, ist die doppelte Lagerhaltung und damit das zweifache Volumen von asymmetrischen Brustprothesen im Vergleich zu symmetrischen Brustprothesen besonders in kleinen Geschäften, die oft sehr beschränkte Lagerungsmöglichkeiten haben, problematisch. Im Handel sind daher asymmetrische Brustprothesen wegen ihrer aufwendigeren Lagerhaltung weniger beliebt als symmetrische Brustprothesen.

Die Aufgabe der Erfindung besteht darin, eine gattungsgemäße Brustprothese so auszubilden, daß sie einen form- und gewichtsmäßig mindestens so günstigen Ausgleich für entferntes Brustgewebe am oberen und achselnahen Brustansatz wie eine asymmetrische Brustprothese schaffen kann, ihre Fähigkeit, wahlweise auf der linken oder rechten Brustseite getragen werden zu können, aber uneingeschränkt beibehält.

Eine Lösung dieser Aufgabe besteht darin, daß die von dem Prothesenrand gebildete geschlossene Linie herzförmig ist und zwei identische Kerben vorgesehen sind, die bezüglich einer den Prothesenkörper in zwei gleich große Hälften unterteilenden Symmetrieebene symmetrisch, in einem zwischen ihnen eingeschlossenen Winkel im Bereich zwischen 30° und 90° angeordnet sind, derart, daß beim Tragen der Prothese nur eine der beiden Kerben im wesentlichen senkrecht zur Körperlängsachse der Trägerin verläuft.

Der Begriff "herzförmig" ist weit auszulegen. Im Grunde genommen soll damit die Form einer symmetrischen Prothese beschrieben werden, die zwei in Abstand voneinander angeordnete Ausläufer hat, von denen der eine zur Schulter weist und der andere zur Achselhöhle weist, wenn die Prothese getragen wird, wobei der Abschnitt des Prothesenrandes zwischen den beiden Ausläufern eine mehr oder weniger konkave Form hat. Selbst ein gerader oder nahezu gerader Verlauf dieses Abschnittes des Prothesenrandes soll nicht bedeuten, daß die gesamte Form der Prothese im Grundriß nicht mehr als "Herzform" bezeichnet werden kann. Wesentlich für die Prothese in Herzform ist, daß sie auf der linken oder rechten Brustseite getragen werden kann, wobei die Prothese bei einem Wechsel der Brustseite nur um 90° gedreht werden muß.

Ein Vorteil der erfindungsgemäßen Brustprothese besteht darin, daß sie die Vorzüge einer asymmetrischen Brustprothese hinsichtlich Tragekomfort und Ausgleichsvermögen mit denen einer symmetrischen Brustprothese hinsichtlich Seitenverwendbarkeit verbindet.

Wie die in der eingangs erwähnten DE 198 38 428 A1 dargestellte Brustprothese, sackt die erfindungsgemäße Brustprothese bei aufrechter Haltung der Trägerin infolge der Kerbwirkung der quer zur Körperlängsachse der Trägerin verlaufenden Kerbe etwas in sich zusammen, wobei der obere Ausläufer leicht nach unten rutscht und der dem Unterbrustbereich entsprechende Teil der Prothese eine den natürlichen Gegebenheiten entsprechende größere Auswölbung erhält. Ebenso wie die aus der DE 198 38 428 A1 bekannte Brustprothese hat die erfindungsgemäße Brustprothese den Vorteil, daß sie sich in liegender Haltung der Trägerin etwas abflacht und infolgedessen auch in liegender Haltung der Trägerin den natürlichen Gegebenheiten entspricht. Darüber hinaus hat die erfindungsgemäße Brustprothese im Vergleich zu anderen bekannten Brustprothesen ohne Kerben den Vorteil eines verbesserten Schwingungsverhaltens und eines geringeren Gewichts.

Im Vergleich zu einer herkömmlichen asymmetrischen Brustprothese hat die erfindungsgemäße Brustprothese den weiteren Vorteil, daß die symmetrischen Ausläufer etwas länger als bei der herkömmlichen asymmetrischen Brustprothese gestaltet werden können, weil der obere Ausläufer bei aufrechter Haltung der Trägerin durch das Zusammensacken der Prothese etwas nach unten rutscht, wogegen der seitliche Ausläufer in der Länge unverändert bleibt und noch weiter zum Achselbereich hin reicht, so daß er dort entferntes Gewebe noch besser als der seitliche Ausläufer einer asymmetrischen Brustprothese ausgleichen kann. Die Verkürzung des oberen Ausläufers der erfindungsgemäßen Brustprothese bei aufrechter Haltung der Trägerin hat den Effekt, daß er nicht aus dem Büstenhalter ragt, was von der Trägerin aus optischen Gründen als angenehm empfunden wird.

Vorzugsweise erstrecken sich die beiden Kerben der Länge nach jeweils auf einer Linie, die jeweils durch eine von zwei gerundeten Ecken der herzförmigen Prothesenrandlinie geht. Dadurch ergibt sich der Vorteil, daß der Übergang zwischen jeder Kerbe und dem Prothesenrand in Längsrichtung der Kerbe besonders sanft ausgeführt werden kann, weil der Prothesenrand an dieser Stelle die größte Entfernung von der jeweiligen Kerbe hat.

Vorzugsweise schließen die beiden Kerben ein im Bereich von 40° bis 60° liegenden Winkel zwischen sich ein.

Bei kleineren Prothesengrößen kann es vorteilhaft sein, wenn sich die beiden Kerben der Länge nach jeweils auf einer Linie erstrecken, die jeweils die herzförmige Prothesenrandlinie an einer Stelle schneidet, die seitlich neben einer von zwei gerundeten Ecken der herzförmigen Prothesenrandlinie liegt. Hierbei kann es weiterhin vorteilhaft sein, daß die beiden Kerben einen Winkel von etwa 90° zwischen sich einschließen. Des weiteren kann es hierbei vorteilhaft sein, wenn sich die beiden Kerben mit ihren inneren Enden auf der Symmetrieebene treffen.

Bei größeren Prothesengrößen kann es von Vorteil sein, wenn zwischen den beiden Kerben mindestens eine weitere Kerbe vorgesehen ist, denn jede weitere Kerbe verbessert das Abflachungsvermögen im Liegen der Protheseträgerin. Jede der beiden Kerben kann in Längsrichtung auch gebogen verlaufen.

Schließlich kann von Vorteil sein, daß zusätzlich zu den beiden Kerben weitere Kerben auf der Rückseite des Prothesenkörpers vorgesehen sind, wobei die weiteren Kerben symmetrisch zu der Symmetrieebene des Prothesenkörpers angeordnet sind.

Die erfindungsgemäße Aufgabe wird auch dadurch gelöst, daß die von dem Prothesenrand gebildete geschlossene Linie herzförmig ist und eine V-förmige Kerbe vorgesehen ist, die bezüglich einer den Prothesenkörper in zwei gleich große Hälften unterteilenden Symmetrieebene symmetrisch ist und einen zwischen ihren beiden Schenkeln eingeschlossenen Öffnungswinkel im Bereich zwischen 30° und 90° hat, wobei im Tragezustand nur einer der beiden Schenkel der Kerbe im wesentlichen senkrecht zur Körperlängsachse der Trägerin verläuft.

Bei größeren Prothesengrößen kann es vorteilhaft sein, wenn zusätzlich zu der V-förmigen Kerbe wenigstens eine weitere Kerbe vorgesehen ist. Auch die weitere Kerbe kann V-förmig sein. Die V-förmige Kerbe und/oder jede weitere V-förmige Kerbe kann jeweils einen gebogenen Abschnitt zwischen den beiden Schenkeln aufweisen. Auch können die beiden Schenkel der V-förmigen Schenkel und/oder die beiden Schenkel jeder weiteren V-förmigen Kerbe in Längsrichtung gebogen verlaufen.

Die erfindungsgemäße Brustprothese kann im Gewicht spürbar reduziert werden, wenn die Silikonkautschukmasse mit einem Füllstoff aus Hohlkugeln vermischt ist, die bezogen auf das Gewicht der Silikonkautschukmasse ein Gewicht von 5 bis 30 Gew.-% haben. Vorzugsweise hat die aus der Silikonkautschukmasse und dem Füllstoff bestehende Mischung eine Viskosität von 2500 bis 8000 mPa·s. Die Hohlkugeln können aus Kunststoff oder Glas sein.

Verschiedene Ausführungsbeispiele der Erfindung sind in den Zeichnungen dargestellt und werden im folgenden näher beschrieben. Es zeigen
- Fig. 1 bis 6: eine Ansicht der Rückseite von symmetrischen, herzförmigen Brustprothesen mit verschiedenen Kerbformen und -anordnungen im unverformten Zustand der Prothese;
- Fig. 7: eine Ansicht der Rückseite einer Brustprothese mit der in Fig. 1 dargestellten Kerbform und -anordnung in getragenem Zustand bei aufrechter Haltung der Trägerin; und
- -Fig. 8: einen Schnitt längs der in Fig. 7 gezeichneten Schnittlinie VIII-VIII.

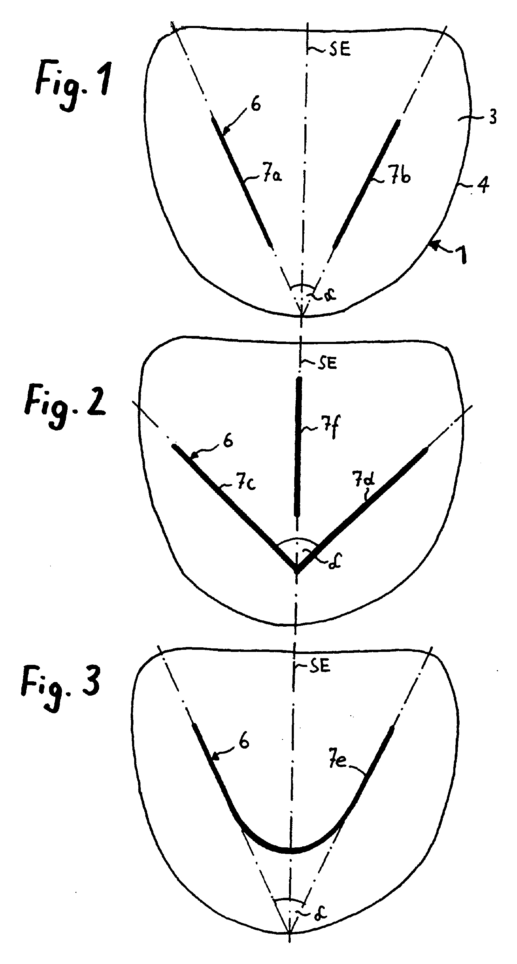

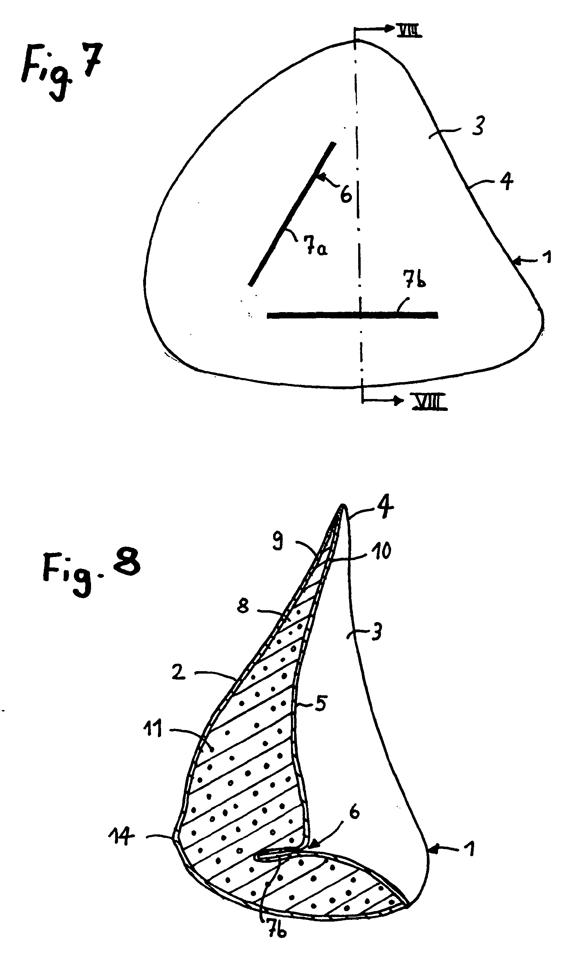

Wie in den Figuren 1, 7 und 8 gezeigt ist, weist eine symmetrische, herzförmige Brustprothese gemäß einem ersten Ausführungsbeispiel einen Prothesenkörper 1 auf, der eine Vorderseite 2, eine Rückseite 3 und einen einer geschlossenen Linie folgenden Prothesenrand 4 hat, an dem die Vorderseite 2 und die Rückseite 3 jeweils enden. Die Vorderseite 2 des Prothesenkörpers 1 hat eine der Form einer natürlichen Brust entsprechende Form. In Fig. 1 ist die Rückseite der Prothese im unverformten Zustand der Prothese dargestellt, in der sie sich zum Beispiel dann befindet, wenn sie in der Mulde des Unterteils der Form liegt, in der sie hergestellt wird. In den Figuren 7 und 8 hingegen ist die Prothese in einem verformten Zustand gezeigt, den sie einnimmt, wenn sie in der rechten Büstenschale eines Büstenhalters getragen wird und die Trägerin sich in einer aufrechten Haltung befindet. Gegenüber dem in Fig. 1 dargestellten Zustand ist die Brustprothese im getragenen Zustand, wie er in Fig. 7 dargestellt ist, um etwa 45° im Uhrzeigersinn gedreht. Würde die Brustprothese in der linken Büstenschale eines Büstenhalters getragen werden, wäre die Brustprothese ausgehend von der in Fig. 1 gezeigten Darstellung etwa um 45° im Gegenuhrzeigersinn gedreht.

Die Rückseite 3 der Prothese ist nach innen gewölbt und bildet infolgedessen eine Vertiefung 5. Wie in den Figuren 1 bis 6 ersichtlich ist, befindet oder befinden sich auf der Rückseite 3 eine bzw. mehrere Kerben 6 in verschiedenen Formen und Anordnungen. Die oder jede Kerbe 6 ist jeweils symmetrisch zu einer dem Prothesenkörper 1 in zwei gleich große Hälften unterteilenden Symmetrieebene SE angeordnet.

Bei dem in Fig. 1 dargestellten Ausführungsbeispiel sind eine linke Kerbe 7a und eine rechte Kerbe 7b vorgesehen, wobei die beiden Kerben 7a und 7b zueinander identisch sind und zwischen sich einen Winkel α von ca. 54° einschließen. Je nachdem, ob die in Fig. 1 dargestellte Brustprothese links oder rechts getragen wird, verläuft die linke Kerbe 7a oder die rechte Kerbe 7b jeweils quer, d.h. im wesentlichen senkrecht zu der Körperlängsachse der Trägerin. In Fig. 7 verläuft die rechte Kerbe 7b im wesentlichen senkrecht zu der Schnittlinie VIII-VIII, die ihrerseits parallel zu der Körperlängsachse der Trägerin verläuft, weil bei der Darstellung in Fig. 7 angenommen wird, daß die Brustprothese auf der rechten Körperseite getragen wird. Die Linien, auf denen die Kerben 7a und 7b liegen, verlaufen durch die gerundeten Ecken der Prothesenrandlinie.

Die Kerben 6 schwächen jeweils den Querschnitt des Prothesenkörpers 1, wodurch der Prothesenkörper 1 im Bereich der Kerben 6 eine Art Scharnier (Filmscharnier) bildet, das sich in Abhängigkeit von der Tragestellung der Brustprothese weitet oder verengt, wenn die jeweilige Kerbe quer zur Körperlängsachse der Trägerin verläuft. Bei der Darstellung in Fig. 7, bei der die Kerbe 7b quer zur Körperlängsachse der Trägerin verläuft und die Trägerin eine aufrechte Haltung einnimmt, hat sich das im Bereich der Kerbe 7b gebildete Scharnier des Prothesenkörpers 1 verengt, und zwar so, wie in Fig. 8 ersichtlich, daß die sich gegenüberliegenden Wände der Kerbe 7b aufeinandersitzen. In liegender Haltung der Trägerin bewegen die sich gegenüberliegenden Wände der Kerbe 7b auseinander, oder anders ausgedrückt, das im Bereich der Kerbe 7b gebildete Scharnier weitet sich und die Prothese flacht sich ab. Bei dem in den Figuren 7 und 8 dargestellten Zustand ist die Prothese etwas in sich zusammengesackt, wobei das Zusammensacken durch das Aneinanderliegen der sich gegenüberliegenden Wände der Kerbe 7b gestoppt wird. Im zusammengesackten Zustand hat sich der in Fig. 7 obere Ausläufer der Prothese etwas nach unten bewegt und der dem Unterbrustbereich entsprechende Teil der Prothese hat sich stärker ausgewölbt.

Das in Abhängigkeit von der Haltung der Trägerin stattfindende Zusammensacken und Abflachen der Prothese, das durch die Kerben ermöglicht wird, spielt sich ähnlich wie bei der aus der DE 198 38 428 A1 bekannten Prothese ab. Auch die Formen der einzelnen Kerben der erfindungsgemäßen Brustprothese sind ähnlich wie diejenigen der einzelnen Kerben der in der DE 198 38 428 A1 offenbarten Brustprothese gestaltet. Der Inhalt der DE 198 38 428 A1 wird deshalb in die vorliegende Anmeldung bzw. das auf die vorliegende Anmeldung erteilten Patents miteinbezogen.

An der Vorderseite 2 der Prothese ist in bekannter Weise eine eine Brustwarze simulierende Noppe 14 ausgebildet. Des weiteren setzt sich der Prothesenkörper 1 hauptsächlich aus einer weichelastischen Silikonkautschukmasse 8 und einem Beutel aus zwei längs des Prothesenrandes 4 dicht miteinander verschweißten, elastisch dehnbaren Kunststoffolien 9, 10 zusammen, wobei die Silikonkautschukmasse 8 in den Beutel hohlraumfrei eingeschlossen ist. Die beiden Kunststoffolien 9, 10 sind Polyurethanfolien.

Bei der in Fig. 2 dargestellten Ausführungsform sind zwei Kerben 7c und 7d vorgesehen, die mit ihren inneren Enden an der Symmetrieebene SE enden und zwischen sich einen Winkel α von ca. 90° einschließen. Die Kerben 7c und 7d liegen jeweils auf einer Linie, die die herzförmige Prothesenrandlinie seitlich unterhalb ihrer gerundeten Ecken schneidet. Eine dritte Kerbe 7f verläuft zwischen den beiden Kerben 7c und 7d auf der Symmetrieebene SE.

Bei der in Fig. 3 dargestellten Ausführungsform ist nur eine einzige Kerbe 7e vorgesehen, die eine V-Form hat, wobei ihre beiden Schenkel durch einen gerundeten mittleren Abschnitt miteinander verbunden sind und einen Winkel α von ca. 54° zwischen sich einschließen.

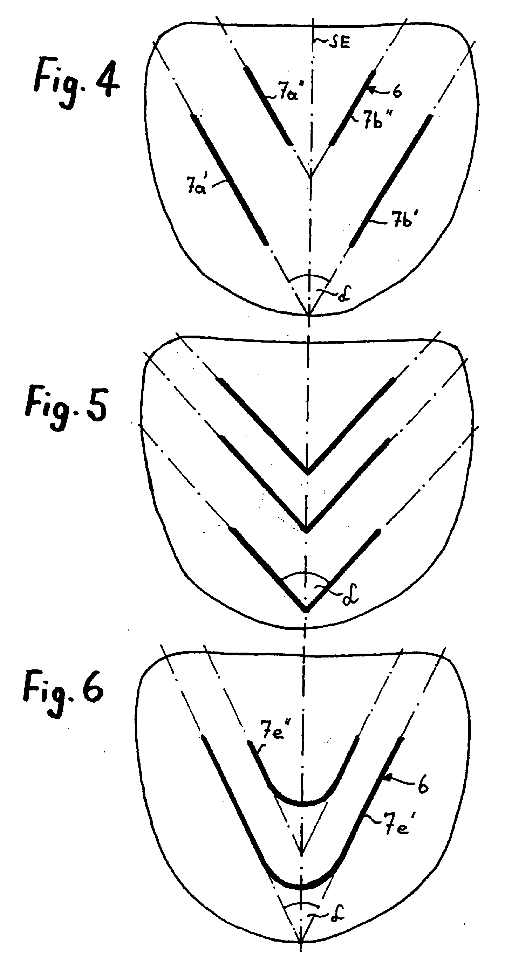

Bei der in Fig. 4 dargestellten Ausführungsform sind zunächst zwei Kerben 7a' und 7b" vorgesehen, die den Kerben 7a und 7b der Ausführungsform von Fig. 1 ähnlich in Form und Anordnung sind. Zusätzlich sind bei der Ausführungsform der Fig. 4 zwei weitere Kerben 7a" und 7b" vorgesehen, die bei der Darstellung in Fig. 4 oberhalb der Kerben 7a' und 7b' angeordnet und jeweils parallel zu der Kerbe 7a' bzw. 7b' sind.

Bei der Ausführungsform der Fig. 5 sind zunächst zwei Kerben 7c' und 7d' vorgesehen, die den Kerben 7c und 7d der Ausführungsform der Fig. 2 ähnlich sind. Zusätzlich sind zwei weitere Kerbenpaare 7c" und 7d" bzw. 7c''' und 7d''' bei der Ausführungsform der Fig. 5 vorgesehen. Die Kerben 7c', 7c" und 7c''' verlaufen parallel zueinander. Die Kerben 7d', 7d" und 7d''' verlaufen ebenfalls parallel zueinander.

Die in Fig. 6 dargestellte Ausführungsform hat zunächst eine Kerbe 7e', die der Kerbe 7e der Ausführungsform der Fig. 3 ähnlich ist. Zusätzlich ist eine Kerbe 7e" oberhalb der Kerbe 7e bei der Ausführungsform der Fig. 6 vorgesehen. Die Kerbe 7e" hat den gleichen V-förmigen Verlauf wie die Kerbe 7e'.

Die Kerbenformen und Kerbenanordnungen sind nicht auf diejenigen der in den Figuren 1 bis 6 dargestellten Ausführungsformen beschränkt. Bei einer Mehrzahl von Kerben können verschiedene Kerbenformen und -anordnungen auch miteinander kombiniert werden. So ist es zum Beispiel auch möglich, daß die in den Figuren 1, 2 und 3 dargestellten Kerbenformen und -anordnungen untereinander kombiniert werden können.

Zu den in den Figuren 1 bis 6 dargestellten Kerben können auch solche hinzugefügt werden, die parallel oder senkrecht zu der Symmetrieebene SE verlaufen. Bei allen Ausführungsformen ist jedoch wesentlich, daß mindestens eine Kerbe oder mindestens ein längerer Abschnitt einer Kerbe quer zur Körperlängsachse der Trägerin verläuft, wenn die Prothese getragen wird.

Allen Ausführungsbeispielen ist gemeinsam, daß die Silikonkautschukmasse mit einem Füllstoff aus Hohlkugeln 11 vermischt ist, die bezogen auf das Gewicht der Silikonkautschukmasse ein Gewicht von 5 bis 30 Gew.-% haben. Die Hohlkugeln 11 können aus Kunststoff oder Glas sein. Die Viskosität der aus der Silikon-kautschukmasse und dem Füllstoff bestehenden Mischung liegt zwischen 2500 und 8000 mPa·s.

## Patentansprüche

1. Symmetrische Brustprothese zum Tragen in der linken oder rechten Büstenschale eines Büstenhalters oder Korseletts nach einer Brustamputation bei Frauen, mit einem Prothesenkörper (1), der eine die Form einer natürlichen Brust aufweisende Vorderseite (2), eine mit wenigstens einer länglichen Kerbe (7a, 7b; 7c, 7d; 7e) versehene konkave Rückseite (3) und einen eine geschlossene Linie bildenden Prothesenrand (4) hat, an dem die Vorderseite (2) und die Rückseite (3) jeweils enden, wobei der Prothesenkörper (1) eine weich-elastische Silikonkautschukmasse (8) aufweist, die in einen Beutel aus längs des Prothesenrandes (4) dicht miteinander verbundenen, elastisch dehnbaren Kunststoffolien (9, 10) hohlraumfrei eingeschlossen ist, **dadurch gekennzeichnet, daß** die von dem Prothesenrand (4) gebildete geschlossene Linie herzförmig ist und zwei identische Kerben (7a, 7b; 7c, 7d) vorgesehen sind, die bezüglich einer den Prothesenkörper (1) in zwei gleich große Hälften unterteilenden Symmetrieebene (SE) symmetrisch, in einem zwischen ihnen eingeschlossenen Winkel (α) im Bereich zwischen 30° und 90° angeordnet sind, derart, daß beim Tragen der Prothese nur eine der beiden Kerben (7a, 7b; 7c, 7d) im wesentlichen senkrecht zur Körperlängsachse der Trägerin verläuft.

2. Symmetrische Brustprothese nach Anspruch 1, **dadurch gekennzeichnet, daß** sich die beiden Kerben (7a, 7b) der Länge nach jeweils auf einer Linie erstrecken, die jeweils durch eine von zwei gerundeten Ecken der herzförmigen Prothesenrandlinie geht.

3. Symmetrische Brustprothese nach Anspruch 2, **dadurch gekennzeichnet, daß** die beiden Kerben (7a, 7b) einen im Bereich von 40° bis 60° liegenden Winkel zwischen sich einschließen.

4. Symmetrische Brustprothese nach Anspruch 1, **dadurch gekennzeichnet, daß** sich die beiden Kerben (7c, 7d) der Länge nach jeweils auf einer Linie erstrecken, die jeweils die herzförmige Prothesenrandlinie an einer Stelle schneidet, die seitlich neben einer von zwei gerundeten Ecken der herzförmigen Prothesenrandlinie liegt.

5. Symmetrische Brustprothese nach Anspruch 4, **dadurch gekennzeichnet, daß** die beiden Kerben (7c, 7d) einen Winkel von etwa 90° zwischen sich einschließen.

6. Symmetrische Brustprothese nach Anspruch 5, **dadurch gekennzeichnet, daß** sich die beiden Kerben (7c, 7d) auf der Symmetrieebene (SE) treffen.

7. Symmetrische Brustprothese nach einem der vorhergehenden Ansprüche, **dadurch gekennzeichnet, daß** zwischen den beiden Kerben (7c, 7d) eine weitere Kerbe (7f) vorgesehen ist.

8. Symmetrische Brustprothese nach einem der vorhergehenden Ansprüche, **dadurch gekennzeichnet, daß** jede der beiden Kerben (7a, 7b; 7c, 7d) in Längsrichtung gebogen verläuft.

9. Symmetrische Brustprothese nach einem der vorhergehenden Ansprüche, **dadurch gekennzeichnet, daß** zusätzlich zu den beiden Kerben (7a', 7b'; 7c', 7d') weitere Kerben (7a", 7b*"*, 7c*"*, 7c*"',* 7d*"*, 7d"') auf der Rückseite (3) des Prothesenkörpers (1) vorgesehen sind, wobei die weiteren Kerben symmetrisch zu der Symmetrieebene (SE) des Prothesenkörpers (1) angeordnet sind.

10. Symmetrische Brustprothese nach dem Oberbegriff des Anspruches 1, **dadurch gekennzeichnet, daß** die von dem Prothesenrand (4) gebildete geschlossene Linie herzförmig ist und eine V-förmige Kerbe (7e) vorgesehen ist, die bezüglich einer den Prothesenkörper (1) in zwei gleich große Hälften unterteilenden Symmetrieebene (SE) symmetrisch ist und einen zwischen ihren beiden Schenkeln eingeschlossenen Öffnungswinkel (α) im Bereich zwischen 30° und 90° hat, wobei im Tragezustand nur einer der beiden Schenkel der V-förmigen Kerbe (7e) im wesentlichen senkrecht zur Körperlängsachse der Trägerin verläuft.

11. Symmetrische Brustprothese nach Anspruch 10, **dadurch gekennzeichnet, daß** wenigstens eine weitere Kerbe (7e") zusätzlich zu der V-förmigen Kerbe (7e) vorgesehen ist.

12. Symmetrische Brustprothese nach Anspruch 11, **dadurch gekennzeichnet, daß** die weitere Kerbe oder die weiteren Kerben jeweils V-förmig ist bzw. sind.

13. Symmetrische Brustprothese nach einem der vorhergehenden Ansprüche, **dadurch gekennzeichnet, daß** die Silikonkautschukmasse (8) mit einem Füllstoff aus Hohlkugeln (11) vermischt ist, die bezogen auf das Gewicht der Silikonkautschukmasse (8) ein Gewicht von 5 bis 30 Gew.-% haben.

14. Symmetrische Brustprothese nach Anspruch 13, **dadurch gekennzeichnet, daß** die aus der Silikonkautschukmasse (8) und dem Füllstoff bestehende Mischung eine Viskosität von 2500 bis 8000 mPa·s hat.

15. Symmetrische Brustprothese nach Anspruch 13 oder 14, **dadurch gekennzeichnet, daß** die Hohlkugeln (11) aus Kunststoff oder Glas sind.

## Claims

1. Symmetrical breast prosthesis for wearing in the left or right cup of a brassiere or corselet following a mastectomy in women, comprising a prosthesis body (1) which has a front (2) having the shape of a natural breast, a concave back (3) provided with at least one elongated notch (7a, 7b; 7c, 7d; 7e), and a prosthesis edge (4) which forms a closed line and at which the front (2) and the back (3) each end, the prosthesis body (1) comprising a flexible silicone rubber material (8) which is enclosed without cavities in a bag comprising elastically extendable plastics films (9, 10) connected tightly to one another along the prosthesis edge (4), **characterized in that** the closed line formed by the prosthesis edge (4) is heart-shaped and two identical notches (7a, 7b; 7c, 7d) are provided which are arranged symmetrically with respect to a plane of symmetry (PS) dividing the prosthesis body (1) into two equal halves, and at an angle (α) in the range between 30° and 90° between them, in such a way that, when the prosthesis is worn, only one of the two notches (7a, 7b; 7c, 7d) is essentially perpendicular to the longitudinal axis of the wearer's body.

2. Symmetrical breast prosthesis according to Claim 1, **characterized in that** the two notches (7a, 7b) each extend in length along a line which in each case passes through one of two rounded corners of the heart-shaped prosthesis contours.

3. Symmetrical breast prosthesis according to Claim 2, **characterized in that** the two notches (7a, 7b) make an angle in the range of 40° to 60° with one another.

4. Symmetrical breast prosthesis according to Claim 1, **characterized in that** the two notches (7c, 7d) each extend in length along a line which in each case intersects the heart-shaped prosthesis contour at a point which is laterally next to one of two rounded corners of the heart-shaped prosthesis contour.

5. Symmetrical breast prosthesis according to Claim 4, **characterized in that** the two notches (7c, 7d) make an angle of about 90° with one another.

6. Symmetrical breast prosthesis according to Claim 5, **characterized in that** the two notches (7c, 7d) meet at the plane of symmetry (PS).

7. Symmetrical breast prosthesis according to any of the preceding Claims, **characterized in that** a further notch (7f) is provided between the two notches (7c, 7d).

8. Symmetrical breast prosthesis according to any of the preceding Claims, **characterized in that** each of the two notches (7a, 7b; 7c, 7d) is curved in the longitudinal direction.

9. Symmetrical breast prosthesis according to any of the preceding Claims, **characterized in that**, in addition to the two notches (7a', 7b'; 7c', 7d'), further notches (7a", 7b" , 7c" , 7c''', 7d" , 7d"') are provided at the back (3) of the prosthesis body (1), the further notches being arranged symmetrically with respect to the plane of symmetry (PS) of the prosthesis body (1).

10. Symmetrical breast prosthesis according to the precharacterizing clause of Claim 1, **characterized in that** the closed line formed by the prosthesis edge (4) is heart-shaped and a V-shaped notch (7e) is provided which is symmetrical with respect to a plane of symmetry (PS) dividing the prosthesis body (1) into two equal halves and has an opening angle (α) in the range between 30° and 90° enclosed between its two limbs, only one of the two limbs of the V-shaped notch (7e) being essentially perpendicular to the longitudinal axis of the wearer's body in the worn state.

11. Symmetrical breast prosthesis according to Claim 10, **characterized in that** at least one further notch (7e") is provided in addition to the V-shaped notch (7e).

12. Symmetrical breast prosthesis according to Claim 11, **characterized in that** the further notch or the further notches is or are V-shaped in each case.

13. Symmetrical breast prosthesis according to any of the preceding Claims, **characterized in that** the silicone rubber material (8) is mixed with a filler comprising hollow spheres (11) which have a weight of from 5 to 30% by weight, based on the weight of the silicone rubber material (8).

14. Symmetrical breast prosthesis according to Claim 13, **characterized in that** the mixture consisting of the silicone rubber material (8) and the filler has a viscosity of from 2500 to 8000 mPa·s.

15. Symmetrical breast prosthesis according to Claim 13 or 14, **characterized in that** the hollow spheres (11) comprise a plastic or glass.

## Revendications

1. Prothèse mammaire symétrique destinée à être portée dans le bonnet gauche ou droit d'un soutien-gorge ou d'un corselet après une ablation de la poitrine d'une femme, comportant un corps de prothèse (1) qui comprend un côté avant (2) présentant la forme d'une poitrine naturelle, un côté arrière (3) concave pourvu d'au moins une entaille allongée (7a, 7b ; 7c, 7d ; 7e) et un bord de prothèse (4) formant une ligne fermée auquel le côté avant (2) et le côté arrière (3) se terminent respectivement, le corps de prothèse (1) présentant une masse de caoutchouc au silicone souple élastique (8) qui est emprisonnée sans cavité dans une poche de feuilles en matière plastique (9, 109) élastiquement extensibles et reliées de façon étanche l'une à l'autre le long du bord de prothèse (4), **caractérisée en ce que** la ligne fermée formée par le bord de prothèse (4) est en forme de coeur et **en ce qu'**il est prévu deux entailles identiques (7a, 7b ; 7c, 7d) qui sont symétriques par rapport à un plan de symétrique (SE) divisant le corps de prothèse (1) en deux moitiés de taille égale, et qui sont agencés sous un angle (α) défini entre eux-mêmes dans la plage de 30° à 90°, de telle sorte que lorsque la prothèse est portée, une seule des deux entailles (7a, 7b ; 7c, 7d) s'étend sensiblement perpendiculairement à l'axe longitudinal du corps de l'utilisatrice.

2. Prothèse mammaire symétrique selon la revendication 1, **caractérisée en ce que** les deux entailles (7a, 7b) s'étendent suivant la longueur sur une ligne respective qui passe chacune par l'un de deux coins arrondis de la ligne de bord de prothèse en forme de coeur.

3. Prothèse mammaire symétrique selon la revendication 2, **caractérisée en ce que** les deux entailles (7a, 7b) définissent entre elles un angle dans la plage de 40° à 60°.

4. Prothèse mammaire symétrique selon la revendication 1, **caractérisée en ce que** les deux entailles (7c, 7d) s'étendent suivant la longueur sur une ligne respective qui recoupe chacune la ligne de bord de prothèse en forme de coeur à un emplacement qui se trouve latéralement à côté de l'un de deux coins arrondis de la ligne de bord de prothèse en forme de coeur.

5. Prothèse mammaire symétrique selon la revendication 4, **caractérisée en ce que** les deux entailles (7c, 7d) définissent entre elles un angle d'environ 90°.

6. Prothèse mammaire symétrique selon la revendication 5, **caractérisée en ce que** les deux entailles (7c, 7d) se rejoignent sur le plan de symétrie (SE).

7. Prothèse mammaire symétrique selon l'une des revendications précédentes, **caractérisée en ce qu'**une autre entaille (7f) est prévue entre les deux entailles (7c, 7d).

8. Prothèse mammaire symétrique selon l'une des revendications précédentes, **caractérisée en ce que** chacune des deux entailles (7a, 7b ; 7c, 7d) s'étend en courbe en direction longitudinale.

9. Prothèse mammaire symétrique selon l'une des revendications précédentes, **caractérisée en ce qu'**en supplément aux deux entailles (7a', 7b' ; 7c', 7d'), d'autres entailles (7a", 7b", 7c", 7c"', 7d", 7d"') sont prévues sur le côté arrière (3) du corps de prothèse (1), les autres entailles étant agencées symétriquement au plan de symétrie (SE) du corps de prothèse (1).

10. Prothèse mammaire symétrique selon le préambule de la revendication 1, **caractérisée en ce que** la ligne fermée formée par le bord de prothèse (4) est en forme de coeur, et **en ce qu'**il est prévu une entaille en forme de V (7e) qui est symétrique par rapport à un plan de symétrique (SE) divisant le corps de prothèse (1) en deux moitiés de taille égale et qui présente un angle d'ouverture (α) enfermé entre ces deux branches dans la plage de 30° à 90°, et dans l'état porté une seule des deux branches de l'entaille en forme de V (7e) s'étend sensiblement perpendiculairement à l'axe longitudinal du corps de l'utilisatrice.

11. Prothèse mammaire symétrique selon la revendication 10, **caractérisée en ce qu'**il est prévu au moins une autre entaille (7e") en supplément à l'entaille en forme de V (7e).

12. Prothèse mammaire symétrique selon la revendication 11, **caractérisée en ce que** l'autre entaille ou les autres entailles est ou sont chacune en forme de V.

13. Prothèse mammaire symétrique selon l'une des revendications précédentes, **caractérisée en ce que** la masse de caoutchouc au silicone (8) est mélangée avec une matière de charge en sphères creuse (11) qui ont un poids de 5 à 30 % par rapport au poids de la masse de caoutchouc au silicone (8).

14. Prothèse mammaire symétrique selon la revendication 13, **caractérisée en ce que** le mélange constitué de la masse de caoutchouc au silicone (8) et de la matière de charge présente une viscosité de 2500 à 8000 mPa·s.

15. Prothèse mammaire symétrique selon la revendication 13 ou 14, **caractérisée en ce que** les sphères creuses (11) sont en matière plastique ou en verre.
